# EUROPÄISCHE PATENTSCHRIFT

(11) **EP 0 454 615 B1**
(45) Veröffentlichungstag und Bekanntmachung des Hinweises auf die Patenterteilung: **14.12.1994**
(21) Anmeldenummer: 91710015.8
(22) Anmeldetag: 26.04.1991
(51) Int. Cl.: A61B 17/32, B26B 17/02

(54) **Zange zum Schneiden chirurgischer Nägel, Drähte od.dgl.**
Forceps for cutting surgical nails, wires or the like
Pince pour couper des clous, des fils ou d'autres articles chirurgicaux similaires

(30) Priorität: 27.04.1990 DE 9004793 U
(43) Veröffentlichungstag der Anmeldung: 30.10.1991
(73) Patentinhaber: JOSEF HEISS - MEDIZINTECHNIK GmbH, D-78532 Tuttlingen (DE)
(72) Erfinder: Heiss, Jürgen Konrad, W-7200 Tuttlingen (DE)
(74) Vertreter: Hiebsch, Gerhard F., Dipl.-Ing.

(56) Entgegenhaltungen:
- EP-A- 0 321 884
- DE-A- 2 216 543
- FR-A- 325 743
- FR-A- 495 124
- FR-A- 495 124
- GB-A- 738 298
- US-A- 1 435 131
- US-A- 2 556 367
- US-A- 2 619 138
- US-A- 2 948 962
- US-A- 4 599 795
- US-A- 4 870 965

## Beschreibung

Die Erfindung betrifft eine Zange -- insbesondere eine Zange zum Schneiden chirurgischer Nägel, Drähte od. dgl. --nach dem Oberbegriff des unabhängigen Patentanspruchs.

Zangen mit einem von zwei Schneiden begrenzten Schneidmaul am Kopfende werden insbesondere in der Orthopädie zum Ablängen von Drähten, Nägeln oder Schraubelementen benötigt. Dabei ist das Verhindern von Spänen od. dgl. Materialpartikel beim Trennvorgang zur Meidung von Infektionen eine hygienische Notwendigkeit.

Die FR-A-495 124 offenbart für Gartenscheren, Stanz- bzw. Lochzangen und vor allem für Blechdosen ein Werkzeug mit den gattungsbildenden Merkmalen, bei dem eine Rolle am freien Ende der Schwenkbacke mit einer gekrümmten Führungskante eines Kulissenstückes zusammenwirkt, welches einem Zahnrad zugeordnet ist. Dessen Zahnung wirkt mit einer Klinke des angelenkten beweglichen Zangenarmes zusammen und wird von dieser um einen beide Zangenarme verbindenden Gelenkbolzen bewegt.

Angesichts dieses Standes der Technik hat sich der Erfinder das Ziel gesetzt, eine Zange der eingangs beschriebenen Art so auszugestalten, daß der Benutzer ein möglichst spanloses Durchtrennen von Materialsträngen oder -profilen auch ohne hohen Kraftaufwand mit einer Hand durchführen kann; die Kraft des Handhabenden soll unabhängig von Materialbeschaffenheit und Strangstärke optimal eingesetzt werden können.

Zur Lösung dieser Aufgabe führt die Lehre des unabhängigen Patentanspruches, die abhängigen Ansprüche bieten Weiterbildungen dazu an.

Erfindungsgemäß ist das Kurvenstück am zweiten Zangenarm vorgesehen, und die Krümmung dieses Kurvenstückes nimmt zum Schneidmaul hin zu. Dank dieser Maßgabe ist es nunmehr möglich, die an der Schneideinrichtung -- beispielsweise einen Klingeneinsatz -- ankommende Kraft durch die Krümmung des Kurvenstückes zu vergleichmäßigen; dazu hat es sich auch als günstig erwiesen, das Kurvenstück als Kulisse für die Rolle an der Zange austauschbar zu gestalten, so daß eine Zange für unterschiedliche Materialien oder Einsatzgebiete verwendet werden kann.

Das Kurvenstück ist vorteilhafterweise Teil eines Führungsabschnittes des zweiten Zangenarmes, und die Bewegungsbahn des Führungsabschnittes ist in Abstand zum Gelenk der Zangenarme angeordnet. Außerdem hat es sich als günstig erwiesen, den führungsabschnitt als Teil eines in Seitenansicht dreiecksförmigen Kopfteiles des zweiten Zangenarmes auszugestalten, wobei eine das Griffende des Zangenarmes verlängernde Dreiecksseite das Kurvenstück aufweist sowie die beiden anderen Dreiecksseiten vom Ende des Kurvenstückes bzw. vom Griffende ausgehen und sich am Gelenk der Zangenarme treffen.

Von besonderer Bedeutung ist die Zuordnung der Gelenke bzw. Achsen; eine das Gelenk zwischen Schwenkbacke und Zangenarm mit der Rollenachse verbindende Gerade soll bevorzugt mit einer durch diese Rollenachse sowie das Gelenk der Zangenarme gelegten Geraden einen Winkel einschließen, der ein wenig größer als 90° ist und insbesondere zwischen 96° und 104° liegt.

Als günstig hat sich zur Begrenzung der Exzenterbewegung eine Anschlagfläche erwiesen, die von einer etwa in einer Senkrechten des Führungsabschnittes durch das Gelenk der Zangenarme verlaufenden Anschlagnase angeboten wird.

Nach einem weiteren Merkmal ist der Abstand des Gelenkes zwischen dem einen Zangenarm und der Schwenkbacke sowie der Zangenstirn kürzer als der Abstand dieses-Gelenkes von der Rolle am Ende der Schwenkbacke.

Zur Verbesserung der Kraftverteilung sowie zur Anpassung der Zange an unterschiedliche Schneidgutdurchmesser kann das Schneidmaul wenigstens zwei Zonen unterschiedlichen Schneidenabstandes aufweist, wobei zumindest eine der Schneiden durch eine quer zur Zangenachse verlaufende Stufenfläche in Schneidenabschnitte unterteilt ist, von denen der dem freien Ende des Schneidenmauls fernere -- bevorzugt -- in geschlossenem Zustand der Zange der gegenüberliegenden Schneide aufliegt. Dank dieser Maßgabe muß nun eine Schneidenpaarung beim Schneidvorgang nicht den gesamten Drahtquerschnitt durchwandern; die -- vorteilhafterweise am freien Ende des Schneidmaules vorgesehene -- Schneidenpaarung mit dem weitesten Abstand zwischen ihren Schneidkanten kerbt den Draht ein, der dann der nächsten Schneidstufe zugeschoben wird; deren Schneidkanten sind einander näher zugeordnet und vermögen mit geringem Kraftaufwand weiter in den Werkstoff einzudringen.

Die Anzahl der Schneidstufen ist von der Beschaffenheit des zu durchtrennenden Werkstoffes bzw. Drahtes od.dgl. abhängig, ihre quer zur Längsachse der Zange erichteten Abstände voneinander bevorzugt gleich groß oder zum Maultiefsten etwas zunehmend. Nach einem anderen Merkmal können die Schneidkanten des einen oder anderen Schneidenpaares -- in geschlossener Stellung des Schneidmaules -- sogar in einem Winkel zur Maullängsachse verlaufen, um bei dieser Schneidstufe völlig parallele Schneidnuten zu erzeugen.

Bei zwei Schneidenabschnitten sowie zwei miteinander fluchtenden Stufenflächen entspricht deren Höhe jeweils etwa einem Viertel der Länge eines Trennschnittes bzw. des Durchmessers eines Drahtes.

Als günstig hat sich zudem erwiesen, die axialen Längen der Schneidenabschnitte etwa gleich groß zu gestalten.

Ebenfalls kann jede Schneidbacke wenigstens eine Auflauffläche aufweisen, die bei geschlossenem Schneidmaul einer Auflauffläche der anderen Schneidbacke anliegt; dank dieser Maßgabe wird ein Übereinanderlaufen der Schneidkanten wirksam vermieden.

Bevorzugt fluchtet die Auflauffläche der Schneidbacke mit deren Schneidkante. Auch hat es sich als günstig erwiesen, wenn die Schneidkante zwischen zwei Auflaufflächen der Schneidbacke verläuft bzw. die Auflaufflächen der Schneidbacke an zwei Hornstücken vorgesehen und diese durch eine Schneide verbunden sind; diese Ausgestaltung erhöht die Stabilität der Schneide bzw. der Schneidkante.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt jeweils in schematisierter Darstellung in
- Fig. 1:: eine Seitenansicht einer Zange für chirurgische Einsätze in Ruhelage;
- Fig. 2:: die Zange der Fig. 1 in Schneidstellung;
- Fig. 3:: eine Schrägsicht auf eine andere Zange in Ruhelage;
- Fig. 4:: die Zange der Fig. 3 in Schneidstellung;
- Fig. 5,7:: jeweils eine weitere Schrägsicht auf eine Zange in Schneidstellung;
- Fig. 6:: ein Schaubild zu einem Schneidvorgang;
- Fig. 8:: eine vergrößerte Sicht auf den Zangenkopf der Fig. 7 mit zwei Schneidbacken;

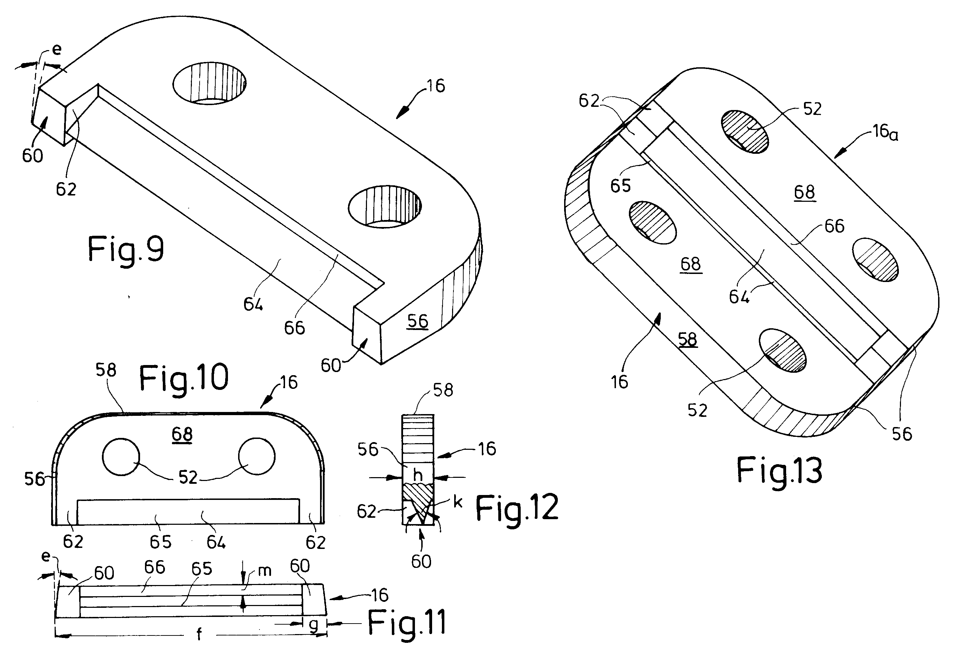

- Fig. 9:: eine Schrägsicht zu Fig. 7, 8 auf eine vergrößerte Schneidbacke;
- Fig. 10:: die verkleinerte Draufsicht auf die Schneidbacke der Fig. 9;
- Fig. 11:: die verkleinerte Frontansicht zu Fig. 9;
- Fig. 12:: eine Seitenansicht zu Fig. 9;
- Fig. 13:: eine Schrägsicht auf die zwei Schneidbacken der Fig. 7, 8;
- Fig. 14:: eine Draufsicht auf einen anderen Zangenkopf mit zwei angedeuteten Schneidbacken bei offenem Schneidmaul;
- Fig. 15,16:: die beiden Schneidbacken der Fig. 14 bei geschlossenem Schneidmaul in verschiedenen Schrägsichten.

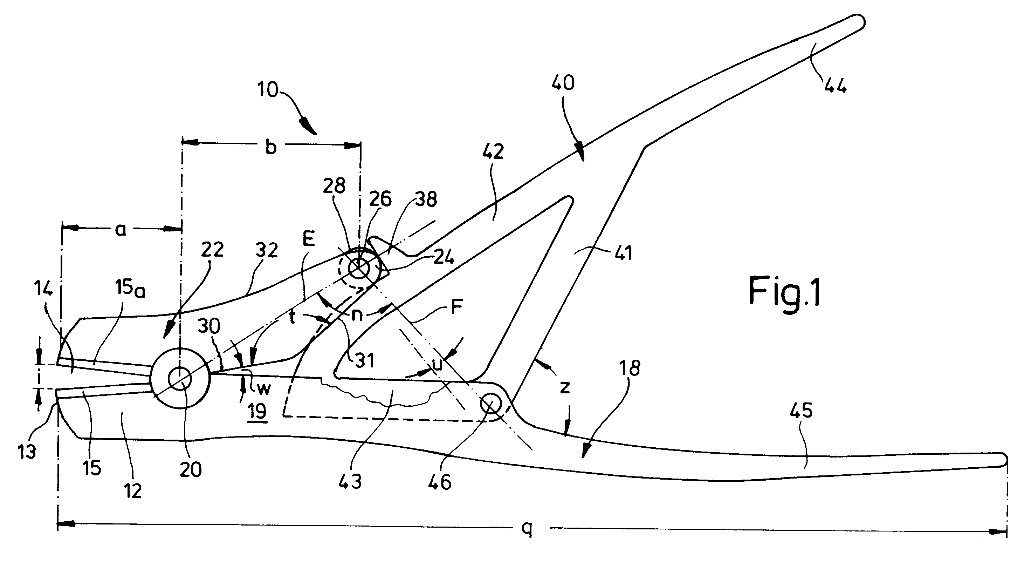

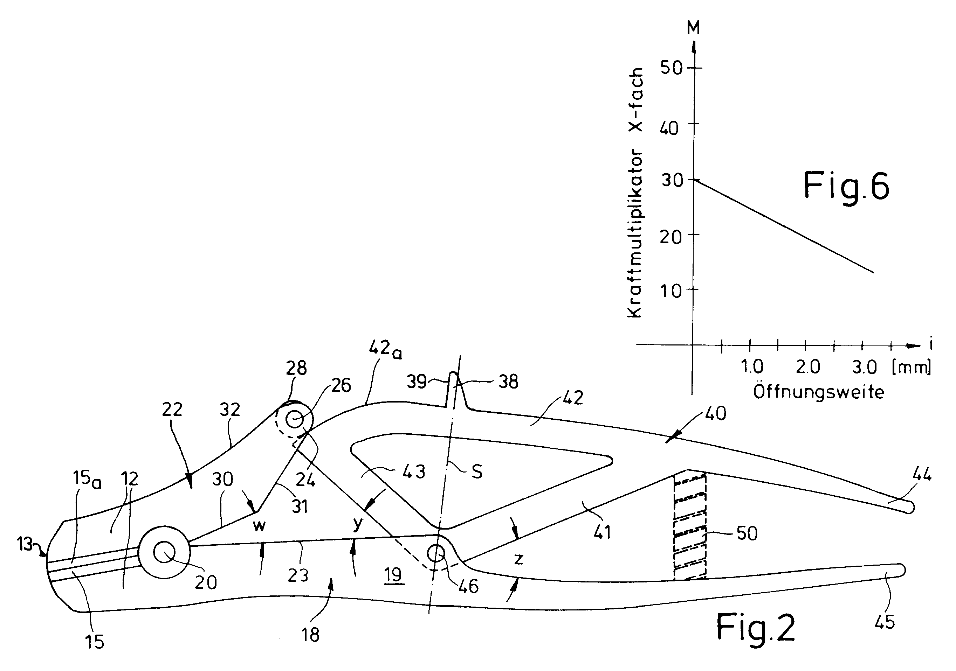

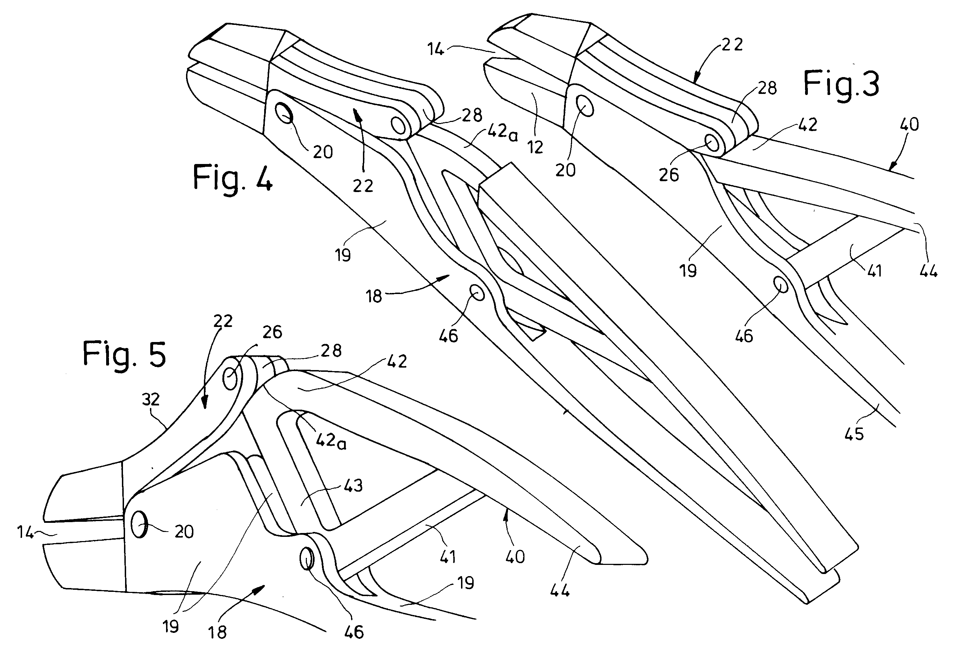

Eine Zange 10 zum im wesentlichen spanlosen Schneiden chirurgischer Nägel, Drähte od.dgl. mittels zweier einander gegenüberliegender, in einem Zangenkopf 12 ein Schneidmaul 14 bestimmender Schneideinsätze 15, 15ₐ weist einen in Fig. 1, 2 unten erkennbaren einstückigen Zangenarm 18 auf, der die Länge q der Zange 10 bestimmt und einends als sich konisch verjüngende Druckwange 12 ausgebildet ist, in welcher der Schneideinsatz 15 sitzt. In den Fig. 3 bis 5 sind die Schneideinsätze 15, 15ₐ aus Gründen der Übersichtlichkeit nicht wiedergegeben.

An den Zangenarm 18 ist am Ende des von ihm angebotenen einen Schneideinsatzes 15 durch ein Kopfgelenk 20 eine mit dem anderen Schneideinsatz 15ₐ versehene Schwenkbacke 22 angelenkt. Diese ist in Seitenansicht nierenartig gestaltet und trägt an ihrem dem Schneideinsatz 15ₐ fernen freien Backenende 24 an einer bolzenartigen Achse 26 eine Rolle 28. Der Abstand a des Kopfgelenkes 20 von der Zangenstirn 13 ist kürzer als sein Abstand b zum Achsbolzen 26 der Rolle 28.

In der in Fig. 1 angedeuteten Ruhelage schließt die Schwenkbacke 22 -- von dem dazu bei einem Abstand i der Schneideinsätze 15, 15ₐ offenen Schneidmaul 14 her gesehen -- hinter dem Kopfgelenk 20 mit dem -- hier von zwei Seitenplatten 19 eines U-Profilabschnittes gebildeten -- Zangenarm 18 einen spitzen Winkel w ein. Der diesen Winkel w begrenzenden Backenkontur 30 folgt in einem stumpfen Winkel t eine Backenkante 31, die mit einer leicht einwärts gekrümmten Außenkante 32 der Schwenkbacke 22 jenes freie Backenende 24 bestimmt.

Nach Fig. 1 liegt die Rolle 28 einer -- bei den Ausführungen der Fig. 3 bis 5 nicht vorhandenen -- Anschlagnase 38 eines dem Zangenarm 18 schwenkbar zugeordneten Druckarmes 40 an, der rollenwärts einen Führungsabschnitt 42 sowie anschließend ein davon nach hinten abragendes Griffende 44 aufweist. Letzteres stellt mit einem Griffende 45 des Zangenarmes 18 den Zangengriff dar, dessen Partner 44, 45 während eines Trennvorganges -- also beim Übergang aus der Ruhelage nach Fig. 1 in die Trennstellung nach Fig. 2 -- sich bei kleiner werdendem Zangen- oder Schließwinkel z einander nähern.

Der Führungsabschnitt 42 des Druckarmes 40 bildet in Seitenansicht mit einem Schrägsteg 41 und einem Endsteg 43 ein einstückiges Dreieck. Schrägsteg 41 und Endsteg 43 treffen sich an einer von einem Gelenkbolzen 46 durchsetzten Lagerstelle; dieser Gelenkbolzen 46 verbindet den Druckarm 40 mit dem Zangenarm 18 so, daß dieser mit dem Schrägsteg 41 jenen Zangenwinkel z begrenzt.

Fig. 1 läßt erkennen, daß eine Verbindungsgerade E von Kopfgelenk 20 und Rollenachse 26 mit einer Verbindungsgeraden F einen Winkel n einschließt, der um einen spitzen Ergänzungswinkel u von etwa 10° größer ist als 90°.

Die Anschlagnase 38 bietetder Rolle 28 eine Anschlagkante 39 an, von der ab jener Führungsabschnitt 42 ein kurzes Stück gerade verläuft und anschließend um den Gelenkbolzen 46 in der Art eines Kurvenstückes 42ₐ als Kulisse für jene Rolle 28 gekrümmt ist.

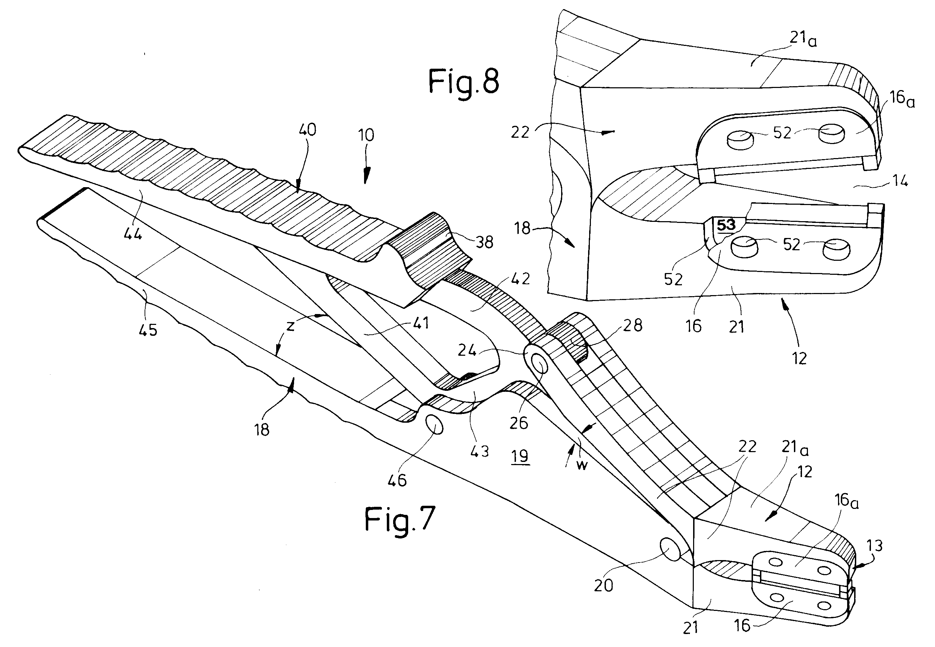

Wird der Zangenwinkel z gegen die Kraft einer bei 50 nur skizzierten Spreizfeder verringert, öffnet sich zum einen zunehmend ein Winkel y zwischen dem Endsteg 43 des Druckarmes 40 sowie einer geraden Innenkante 23 des Zangenarms 18. Zum anderen fährt die Rolle 28 jenes Kurvenstück 42ₐ ab, dessen Krümmung mit zunehmendem Abstand zur Anschlagfläche 39 zunimmt und somit die auf die Schneideinsätze 15, 15ₐ wirkende Kraft gleichmäßig erhöht, wie dies Fig. 6 am Verlauf einer Geraden G über der Öffnungsweite i des Schneidmauls 14 erkennen läßt; vertikal dazu ist in Fig. 6 der x-fache Kraftmultiplikator M aufgetragen.

Die Fig. 3 und 4 machen besonders deutlich, daß das Dreieck 41, 42, 43 zwischen den Seitenplatten 19 des Zangenarmes 18 geführt lagert und sich zur Ruhelage hin in das U-Profil absenkt (Fig. 3).

Die Schneideinsätze 15, 15ₐ werden so durch ein Exzentersystem bewegt, das die erwähnte Vergleichmäßigung der Kraft erlaubt.

In Fig. 7 sind zwei Schneidbacken 16, 16ₐ in Druckbacken 21, 21ₐ zu erkennen. Beide Schneidbacken 16, 16ₐ einer Länge f von etwa 22 mm und einer Höhe h von etwa 2,2 mm sind in entsprechend geformten Ausnehmungen 52 ihrer jeweiligen Druckwange 21, 21ₐ festgelegt und zwar mittels nicht wiedergegebener Schrauben, welche Bohrungen 54 der Schneidbacken 16, 16ₐ durchsetzen und in einem Anschlagboden 53 der Ausnehmung 52 festliegen. In einem spitzen Winkel e geneigte Seitenwände 56 der i.w. rechteckigen Schneidbacke 16 gehen gerundet in einen Backenrücken 58 über, dessen Fläche ihrerseits geringfügig geneigt ist. Die Seitenwände 56 enden jeweils an einer Auflauffläche 60, deren Breite g etwa ihrer Höhe h entspricht.

Innerhalb zweier zinnenartig abragender, die Auflaufflächen 60 anbietender Hornstücke 62 der Schneidbacke 16, 16ₐ erstreckt sich eine Schneide 64, die unter Bildung eines Wandstückes 66 in Abstand m zur Backenoberfläche 68 am Schneidbackenkörper ansetzt und sich bis zu der die Auflaufflächen 60 verbindenden Schneidkante 65 abwärts neigt. Es wird deutlich, daß die Auflaufflächen eine wesentliche Verminderung der Keilwinkel k der Schneiden 64 -- ohne die Gefahr eines Übereinanderscherens der Schneidbacken 16, 16ₐ -- gestatten.

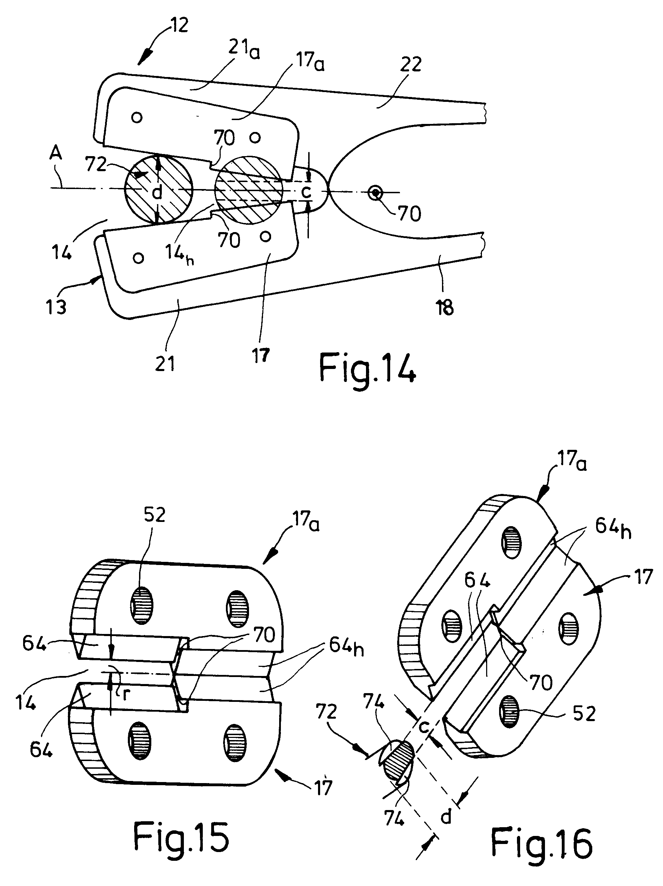

Fig. 14 verdeutlicht eine chirurgische Zange 10ₐ, deren Druckwangen 21, 21ₐ -- und damit ihre Schneidwangen 17, 17ₐ -- um einen konstruktiven Drehpunkt 70 relativ zueinander geschwenkt werden können.

Die Schneidbacken 17, 17ₐ sind unter Bildung quer zur Zangenlängsachse A verlaufender Stufenflächen 70 mehrfach -- im gewählten Ausführungsbeispiel einmal -- abgestuft, d.h. es entstehen hier zwischen den Schneiden 64 und 64ₕ zwei Schneidmaulabschnitte 14, 14ₕ. Deren von der Zangenstirn 13 ausgehender dient zum Anschneiden eines bei 72 erkennbaren Drahtes des Durchmessers d unter Erzeugung eines Kerbenpaares 74 des gelenkwärts leicht abnehmenden Abstandes c; dann wird der Draht 72 zur nächsten Maulstufe 14ₕ verschoben, um zwischen deren einander näher stehenden Schneiden 64ₕ durchtrennt zu werden.

Die beschriebene stufenförmige Schneidbackenausführung ermöglicht ein Durchtrennen größerer Durchmesser von beispielsweise 5 mm in zwei Trennstufen, wobei in jeder das paar der Schneiden 64 bzw. 64ₕ nur einen Teil des Trennweges d tätig sind, dessen Maß durch den Abstand r der benachbarten Schneiden 64 und 64ₕ bestimmt ist, der in Fig. 14 etwa dem halben Maß d entspricht.

Nicht dargestellt ist eine Ausführung, bei der die Schneidkanten des vorderen Schneidmaulabschnittes 14 zur Zangenlängsachse A --als Schneidmaullängsachse -- etwas so geneigt verlaufen, daß sie einander am freien Ende des Abschnittes 14 geringfügig näher liegen als im Schneidmaulinneren; es wird dadurch erreicht, daß die damit erzeugten Kerben 74 zueinander parallele Kerbentiefste oder Kerbenböden aufweisen.

## Patentansprüche

1. Zange, insbesondere zum Schneiden chirurgischer Nägel, Drähte od. dgl., mit einem von zwei Schneideinrichtungen (15,15a) begrenzten Schneidmaul (14) am Kopfende der Zange, die andernends zwei gegen eine Rückstellkraft in eine Schneidstellung der Schneideinrichtungen zusammenführbare Griffenden (44,45) von aneinandergelenkten Zangenarmen (18,40) aufweist, wobei eine der Schneideinrichtungen (15a) an einer Schwenkbacke (22) vorgesehen ist, welche an einem die andere Schneideinrichtung (15) tragenden ersten Zangenarm (18) angelenkt ist sowie mit ihrem freien Ende (24) einem Kurvenstück (42a) anliegt,
dadurch gekennzeichnet,
daß das Kurvenstück (42a) am zweiten Zangenarm (40) vorgesehen ist sowie die Krümmung des Kurvenstücks (42a) zum Schneidmaul (14) hin zunimmt.

2. Zange nach Anspruch 1, dadurch gekennzeichnet, daß als Wegbegrenzung für das freie Ende (24) der Schwenkbacke (22) bzw. für eine an sich bekannte Rolle (28) an der Schwenkbacke (22) eine Anschlagfläche (38) am Ende des von ihm/ihr berührten Kurvenstückes (42a) vorgesehen ist.

3. Zange nach Anspruch 1 oder 2, dadurch gekennzeichnet, daß das Kurvenstück (42a) Teil eines Führungsabschnittes (42) des zweiten Zangenarmes (40) und die Bewegungsbahn des Führungsabschnittes in Abstand zum Gelenk (46) der Zangenarme (18,40) angeordnet ist.

4. Zange nach Anspruch 2 oder 3, dadurch gekennzeichnet, daß die Anschlagfläche (39) von einer Anschlagnase (38) gebildet ist, die etwa in einer Senkrechten des Führungsabschnittes (42) durch das Gelenk (46) der Zangenarme (18, 40) verläuft.

5. Zange nach Anspruch 3 oder 4, dadurch gekennzeichnet, daß der Führungsabschnitt (42) Teil eines in Seitenansicht dreiecksförmigen Kopfteiles (41 bis 43) des zweiten Zangenarmes (40) ist, wobei eine das Griffende (44) des Zangenarmes (40) verlängernde Dreiecksseite das Kurvenstück (42a) und gegebenenfalls die Anschlagfläche (39) aufweist sowie die beiden anderen Dreiecksseiten (41, 43) vom Ende des Kurvenstückes (42a) bzw. vom Griffende (44) ausgehen und sich am Gelenk (46) der Zangenarme (18, 40) treffen.

6. Zange nach einem der Ansprüche 2 bis 4, dadurch gekennzeichnet, daß eine das Gelenk (20) zwischen Schwenkbacke (22) und Zangenarm (18) mit der Rollenachse (26) verbindende Gerade (E) mit einer durch diese Rollenachse sowie das Gelenk (46) der Zangenarme (18, 40) gelegten Geraden (F) einen Winkel (n) einschließt, der ein wenig größer als 90° ist, insbesondere durch einen Winkel (n) zwischen 96° und 104°.

7. Zange nach wenigstens einem der Ansprüche 1 bis 5, dadurch gekennzeichnet, daß der Abstand (a) des Gelenkes (20) zwischen dem einen Zangenarm (18) und der Schwenkbacke (22) sowie der Zangenstirn (13) kürzer ist als der Abstand (b) dieses Gelenkes von der Rolle (28), und/oder, daß das Kurvenstück (42a) am Zangenarm (40) austauschbar angebracht ist.

8. Zange nach wenigstens einem der Ansprüche 1 bis 7, dadurch gekennzeichnet, daß das Schneidmaul (14) wenigstens zwei Zonen (14, 14a) unterschiedlichen Schneidenabstandes aufweist, wobei zumindest eine der Schneiden durch eine quer zur Zangenlängsachse (A) verlaufende Stufenfläche (70) in Schneidenabschnitte (64, 64h) unterteilt ist, von denen der dem freien Ende (13) des Schneidenmauls fernere bevorzugt in geschlossenem Zustand der Zange (10a) der gegenüberliegenden Schneide (64, 64h) aufliegt.

9. Zange nach wenigstens einem der Ansprüche 1 bis 7, dadurch gekennzeichnet, daß die Zangenlängsachse (A) eine Symmetriegerade für die Schneiden (15, 15a, 64, 64h) bildet.

10. Zange nach Anspruch 8 oder 9, gekennzeichnet durch zwei Schneidenabschnitte (64, 64h) sowie zwei miteinander fluchtenden Stufenflächen (70), deren Höhe (r) jeweils etwa einem Viertel der Länge (d) eines Trennschnittes in einem Draht (72) od.dgl. entspricht, wobei gegebenenfalls die axialen Längen der Schneidenabschnitte (64, 64a) etwa gleich sind.

11. Zange nach wenigstens einem der Ansprüche 1 bis 10, dadurch gekennzeichnet, daß jede Schneidbacke (16, 16a) wenigstens eine Auflauffläche (60) aufweist, welche bei geschlossenem Schneidmaul (14) einer Auflauffläche der anderen Schneidbacke (16a, 16) anliegt, wobei gegebenenfalls die Auflauffläche der Schneidbacke mit deren Schneidkante (65) fluchtet.

12. Zange nach Anspruch 11, dadurch gekennzeichnet, daß die Schneidkante (65) zwischen zwei Auflaufflächen (60) der Schneidbacke (16, 16a) verläuft, und/oder, daß die Auflaufflächen (60) der Schneidbacke (16, 16a) an zwei Hornstücken (62) vorgesehen und diese durch eine Schneide (64) verbunden sind.

13. Zange nach wenigstens einem der Ansprüche 1 bis 12, dadurch gekennzeichnet, daß die Schneidbacke (15, 16, 17) auswechselbar in einer Druckbacke (21, 21a) festgelegt ist, und/oder, daß sich die Schneidbacke gegen einen Anschlagboden (53) einer Ausnehmung (52) einer Druckbacke (21, 21a) abstützt.

14. Zange nach wenigstens einem der Ansprüche 1 bis 13, dadurch gekennzeichnet, daß die Schneidkanten (65) wenigstens eines der Schneidkantenpaare zur Längsachse des Schneidmaulabschnittes (14) etwas geneigt verlaufen und an der dem freien Ende der Zange nahen Ende des Schneidmaulabschnittes einander näher liegen als am anderen Ende.

## Claims

1. Forceps, particularly for cutting surgical nails, wires or the like, comprising a cutting mouth (14) defined by two cutting means (15, 15a) at the head end of the forceps, and provided at the other end with two gripping ends (44, 45) of forcep arms (18, 40) hinged together which can be moved towards one another against a restoring force into a cutting position of the cutting means, one of the cutting means (15a) being provided on a swivel jaw (22) which is hinged on to a first forcep arm (18) carrying the other cutting means (15) and abuts via its free end (24) against a curved piece (42a), characterised in that the curved piece (42a) is provided on the second forcep arm (40) and the curvature of the curved piece (42a) increases towards the cutting mouth (14).

2. Forceps according to claim 1, characterised in that a stop face (38) to limit the travel of the free end (24) of the swivel jaw (22) or of a roller (28) known per se on the swivel jaw (22) is provided at the end of the curved piece (42a) contacted by it.

3. Forceps according to claim 1 or claim 2, characterised in that the curved piece (42a) is part of a guide portion (42) of the second forcep arm (40) and the path of motion of the guide portion is arranged at a distance from the joint (46) of the forcep arms (18, 40).

4. Forceps according to claim 2 or claim 3, characterised in that the stop face (39) is formed by a stop boss (38) which extends approximately in a vertical of the guide portion (42) through the joint (46) of the forcep arms (18, 40).

5. Forceps according to claim 3 or claim 4, characterised in that the guide portion (42) is part of a head part (41 to 43) of the second forcep arm (40) which is triangular when viewed from the side, one side of the triangle extending the gripping end (44) of the forcep arm (40) having the curved piece (42a) and optionally the stop face (39) and the other two sides of the triangle (41, 43) departing from the end of the curved piece (42a) or from the gripping end (44) and meeting at the joint (46) of the forcep arms (18, 40).

6. Forceps according to one of claims 2 to 4, characterised in that a straight line (E) connecting the joint (20) between the swivel jaw (22) and the forcep arm (18) to the roller axis (26) includes an angle (n) with a straight line (F) extending through this roller axis and the joint (46) of the forcep arms (18, 40) which is slightly greater than 90°, particularly through an angle (n) of between 96° and 104°.

7. Forceps according to at least one of claims 1 to 5, characterised in that the distance (a) between the joint (20) between one forcep arm (18) and the swivel jaw (22) and the end (13) of the forceps is shorter than the distance (b) between this joint and the roller (28) and/or that the curved piece (42a) is replaceably mounted on the forcep arm (40).

8. Forceps according to at least one of claims 1 to 7, characterised in that the cutting mouth (14) has at least two zones (14, 14a) with a different cutting distance, at least one of the cutters being divided by a step face (70) extending transversely to the longitudinal axis (A) of the forceps into cutting portions (64, 64h), the cutting portion further away from the free end (13) of the cutting mouth preferably abutting against the opposing cutters (64, 64h) in the closed position of the forceps (10a).

9. Forceps according to at least one of claims 1 to 7, characterised in that the longitudinal axis (A) of the forceps forms a line of symmetry for the cutters (15, 15a, 64, 64h).

10. Forceps according to claim 8 or claim 9, characterised by two cutting portions (64, 64h) and two step faces (70) aligned with one another, the height (r) of which corresponds to approximately a quarter of the length (d) of a separating cut in a wire (72) or the like, the axial lengths of the cutting portions (64, 64a) optionally being approximately equal.

11. Forceps according to at least one of claims 1 to 10, characterised in that each cutting jaw (16, 16a) has at least one abutting surface (60) which abuts against an abutting surface of the other cutting jaw (16a, 16) when the cutting mouth (14) is closed, the abutting surfaces of the cutting jaw optionally being aligned with the cutting edge (65) thereof.

12. Forceps according to claim 11, characterised in that the cutting edge (65) extends between two abutting surfaces (60) of the cutting jaw (16, 16a) and/or that the abutting surfaces (60) of the cutting jaw (16, 16a) are provided on two horn pieces (62) and these are connected together by means of a cutter (64).

13. Forceps according to at least one of claims 1 to 12, characterised in that the cutting jaw (15, 16, 17) is replaceably fixed in a pressure jaw (21, 21a) and/or that the cutting jaw is supported against a stop base (53) of a recess (52) in a pressure jaw (21, 21a).

14. Forceps according to at least one of claims 1 to 13, characterised in that the cutting edges (65) of at least one of the cutting edge pairs extend at a slight incline relative to the longitudinal axis of the cutting mouth portion (14) and are closer together at the end of the cutting mouth portion close to the free end of the forceps than at the other end.

## Revendications

1. Pince, en particulier pour couper des clous et fils chirurgicaux ou similaires, comportant, sur l'extrémité de tête de la pince, une bouche de coupe (14), qui est limitée par deux dispositifs de coupe (15, 15a) et qui présente, à l'autre extrémité, deux extrémités de manche (44, 45) de bras de pince (18,40) articulés entre eux, les extrémités de manche pouvant, contre une force de rappel, être réunies dans une position de coupe des dispositifs de coupe, et un des dispositifs de coupe (15a) étant prévu au niveau d'une mâchoire pivotante (22) qui est articulée sur un premier bras de pince (18) portant l'autre dispositif de coupe (15) et qui appuie, par son extrémité libre (24), sur une pièce courbe (42a),
caractérisée en ce que
la pièce courbe (42a) est prévue sur le second bras de pince (40) et en ce que la courbure de la pièce courbe (42a) augmente quand on se rapproche de la bouche de coupe (14).

2. Pince selon la revendication 1, caractérisée en ce que, à titre de limitation de course destinée à l'extrémité libre (24) de la mâchoire pivotante (22) ou à un galet (28) connu en lui-même sur la mâchoire pivotante (22), on prévoit une surface de butée (39) à l'extrémité de la pièce courbe (42a) qui est touchée par cette extrémité ou ce galet.

3. Pince selon la revendication 1 ou 2, caractérisée en ce que la pièce courbe (42a) fait partie d'un segment de guidage (42) du second bras de pince (40) et en ce que le chemin de déplacement du segment de guidage est disposé à l'écart de l'articulation (46) des bras de pince (18, 40).

4. Pince selon la revendication 2 ou 3, caractérisée en ce que la surface de butée (39) est constituée d'un bec de butée (38) qui est disposé à peu près dans une perpendiculaire du segment de guidage (42) à travers l'articulation (46) des bras de pince (18, 40).

5. Pince selon la revendication 3 ou 4, caractérisée en ce que le segment de guidage (42) fait partie d'une pièce de tête (41 à 43), de forme triangulaire en vue de côté, du second bras de pince (40), où un côté de triangle prolongeant l'extrémité de manche (44) du bras de pince (40) présente la pièce courbe (42a) et éventuellement la surface de butée (39) tandis que les deux autres côtés du triangle (41, 43) partent de l'extrémité de la pièce courbe (42a) et de l'extrémité de manche (44) et se rencontrent au niveau de l'articulation (46) des bras de pince (18, 40).

6. Pince selon l'une des revendications 2 à 4, caractérisée en ce qu'une droite (E) reliant l'articulation (20) entre la mâchoire pivotante (22) et le bras de pince (18) à l'axe de galet (26) forme, avec une droite (F) traversant cet axe de galet ainsi que l'articulation (46) des bras de pince (18, 40), un angle (n) qui est légèrement supérieur à 90°, en particulier un angle (n) compris entre 96° et 104°.

7. Pince selon au moins l'une des revendications 1 à 5, caractérisée en ce que l'écart (a) de l'articulation (20) entre un bras de pince (18) et la mâchoire pivotante (22) ainsi que la face de pince (13) est plus court que l'écart (b) de cette articulation par rapport au galet (28) et/ou en ce que la pièce courbe (42a) sur le bras de pince (40) est disposée de façon à pouvoir être remplacée.

8. Pince selon au moins l'une des revendications 1 à 7, caractérisée en ce que la bouche de coupe (14) présente au moins deux zones (14, 14a) présentant un écart de tranchant différent, au moins un des tranchants étant divisé en segments de tranchant (64, 64h) par une surface en gradins (70) oblique par rapport à l'axe longitudinal de pince (A), segments de coupe dont celui qui est le plus éloigné de l'extrémité libre (13) de la bouche de coupe est de préférence exposé au tranchant opposé (64, 64h) lorsque la pince (10a) est fermée.

9. Pince selon au moins l'une des revendications 1 à 7, caractérisée en ce que l'axe longitudinal de pince (A) forme une droite de symétrie pour les tranchants (15, 15a, 64, 64h).

10. Pince selon la revendication 8 ou 9, caractérisée par deux segments de tranchant (64, 64a) ainsi que par deux surfaces en gradins (70) quiaffleurent l'une avec l'autre et dont la hauteur (r) correspond à chaque fois à environ un quart de la longueur (d) d'une coupe effectuée dans un fil (72) ou élément similaire, les longueurs axiales des segments de tranchant (64, 64h) étant éventuellement à peu près égales.

11. Pince selon au moins l'une des revendications 1 à 10, caractérisée en ce que chaque mâchoire de coupe (16, 16a) présente au moins une surface ascendante (60) qui, lorsque la bouche de coupe (14) est fermée, appuie contre une surface ascendants de l'autre mâchoire de coupe (16a, 16), la surface ascendante de la mâchoire de coupe affleurant éventuellement avec son arête de coupe (65).

12. Pince selon la revendication 11, caractérisée en ce que l'arête de coupe (65) s'étend entre deux surfaces ascendantes (60) de la mâchoire de coupe (16, 16a) et/ou en ce que les surfaces ascendantes (60) de la mâchoire de coupe (16, 16a) sont prévues sur deux pièces en corne (62) et que celles-ci sont reliées par un tranchant (64).

13. Pince selon au moins l'une des revendications 1 à 12, caractérisée en ce que la mâchoire de coupe (15, 16, 17) est maintenue, de façon amovible, dans une mâchoire de pression (21, 21a) et/ou en ce que la mâchoire de coupe appuie contre un fond de butée (53) d'un évidement (52) d'une mâchoire de pression (21, 21a).

14. Pince selon au moins l'une des revendications 1 à 13, caractérisée en ce que les arêtes de coupe (65) d'au moins une des paires d'arêtes de coupe sont légèrement inclinées par rapport à l'axe longitudinal du segment de bouche de coupe (14) et sont, à l'extrémité de segment de bouche de coupe proche de l'extrémité libre de la pince, plus rapprochées l'une de l'autre que sur l'autre extrémité .
